# EUROPEAN PATENT SPECIFICATION

(11) **EP 0 487 774 B1**
(45) Date of publication and mention of the grant of the patent: **26.10.1994**
(21) Application number: 90122804.9
(22) Date of filing: 29.11.1990
(51) Int. Cl.: A61K 9/20

(54) **A direct tabletting auxiliary**
Hilfsträger für direkte Verpressung
Auxiliaire de compression directe

(43) Date of publication of application: 03.06.1992
(73) Proprietor: WEI MING PHARMACEUTICAL MFG. CO. LTD., Taipei, Taiwan R.O.C. (TW)
(72) Inventor: Lang, Siegfried, Dr., W-6700 Ludwigshafen (DE); Yeh, Ta-Shuong, Taipei (TW)
(74) Representative: Cresswell, Thomas Anthony

(56) References cited:
- EP-A- 0 140 203
- EP-A- 0 192 173
- EP-A- 0 265 951
- CHEM. PHARM. BULL., vol. 32, no. 2, 1984, pages 665-669; E. FENYVESI et al.:"Properties of Cyclodextrin Polymers as a tabletting aid"

## Description

The present invention relates to a direct tabletting auxiliary based on a tablet filler, preferably microcrystalline cellulose (MCC) with a binder, preferably β-cyclodextrin, the auxiliary having been prepared by a wet mixing process.

Currently used for direct tabletting, i.e. the dry mixing of tabletting auxiliary and active substance and compression, in the pharmaceutical industry are a number of carrier materials such as cellulose powder, di-calcium phosphate, sorbitol, MCC, dextrose, lactose or lactose/cellulose.

The main requirements to be met by a direct tabletting auxiliary of this type are: good flowability, good compressibility under low pressure, and high loading capacity.

The tablets produced therewith should have satisfactory hardness, low friability and good disintegration and dissolution properties.

These requirements are only partly met by commercial products.

EP-A-192173 discloses a direct tabletting auxiliary comprising (a) 88 to 96 weight percent of a filler of lactose or lactose together with a similar amount of other carrier material; (b) 2 to 6 weight percent of polyvinylpyrrolidone and (c) 2 to 10 percent by weight of insoluble polyvinylpyrrolidone and prepared by spray drying, spray granulation or wet granulation.

EP-A-265951 describes a granular directly compressible tricalciumphosphate having a particle size sufficient for efficient direct compression tabletting which can be prepared by densifying compacting tricalciumphosphate to a particle size sufficient for wet granulation.

EP-A-140203 describes a pharmaceutical preparation in solid form comprising acetylsalacylic acid characterized in that it comprises an additive β-cyclodextrin.

Fenyvesi *et al* Chem. Pharm. Bul. 32 (2) 665-669 (1984) describes the properties of cyclodextrin polymer as a tabletting aid, and in particular as a disintegrant.

The method of direct tabletting is of particular interest to the pharmaceutical industry because, on the one hand, it allows stress-free processing of active substances and, on the other hand, the costs of processing and producing tablets are lower.

Microcrystalline cellulose has been used as auxiliary for direct tabletting for many years world-wide (e.g. as Avicel® PH 101 and PH 102), and is described *inter alia* in USP XXII/NF XVII, page 1915, to which reference is made.

It is an object of the present invention to propose a direct tabletting auxiliary which meets the said requirements and, moreover, makes a higher loading capacity possible, which means that tablets with a content of active substance of from 70 to 75% can be produced, and addition of a disintegrant is unnecessary in most cases.

We have found that this object is achieved by a novel direct tabletting auxiliary containing on a weight basis
A) 60 to 98%, preferably 80 to 98%, of a filler comprising microcrystalline cellulose or cellulose powder, preferably microcrystalline cellulose, and
B) 2 to 40%, preferably 2 to 20%, of a binder comprising α-, β- or γ-cyclodextrin, preferably β-cyclodextrin, were A and B are mixed with water or water/alcohol by using a wet mixing process, in particular a wet granulation process, spray granulation process or spray drying and then simultaneous or subsequent drying.

The binder B comprises α-, β- or γ-cyclodextrin, preferably β-cyclodextrin, as marketed under the name KLEPTOSE® by Roquette.

The auxiliary is formed by a wet mixing process by which is meant a process in which the components A and B which have been moistened with water or an alcohol/water mixture, i.e. usually with a quantity of water which is insufficient to dissolve the binder completely, are uniformly mixed in a mixing apparatus and simultaneously or subsequently dried.

The procedure for spray granulation is, for example, such that a mixture of MCC and the binder is introduced into the fluidized bed and, with the temperature slightly elevated, e.g. at from 40 to 60°C, sprayed with water, resulting in a dried product.

Wet granulation entails, for example, mixing MCC with the binder in a suitable mixer, pouring water in while continuing to stir, and drying the moist material after it has been passed through a screen, or moistening the tablet filler with a solution or suspension of the binder in water. The moist material is then screened and dried.

The spray drying is usually carried out in such way that an aqueous suspension of the tablet filler A and component B is sprayed in a suitable spraying apparatus concurrently or countercurrently with the drying air at elevated temperatures, eg. at an inlet temperature of the drying air or up to 120°C.

The said processes are expediently used, starting from finely powdered MCC, to prepare a powder with a narrow particle size distribution of, for example, from about 25 to 250 µm with from 60 to 70% in the range from 40 to 75 µm. Of the methods which have been mentioned, wet granulation is preferred and gives particularly good results.

The mixtures according to the invention which are obtained have excellent tabletting properties and are distinguished from known direct tabletting auxiliaries by, in particular, good flowability, good compressibility under low pressure and excellent disintegration properties with high hardness and low friability of the tablets.

The examples which follow describe both the preparation of the mixtures according to the invention and the production of tablets, comparing with direct tabletting auxiliaries which have been prepared by physical mixing.

Examples
I

| | |
|---|---|
| MCC wet (51% solid content) | 5.0 kg |
| β-Cyclodextrin | 670 g |
| Distilled water | 2.9 kg |

Suspend 670 g β-cyclodextrin in 2.9 kg water and add the suspension to the wet MCC in a kneader. After 5 minutes intensive blending pass through a sieve (0.5 mm) and dry the material at 80°C. After drying pass it again through a sieve (0.250 mm) (water content below 6%).
II

| | |
|---|---|
| MCC wet (51% solid content) | 5.0 kg |
| β-Cyclodextrin | 210 g |
| Ethanol 95% | 2.0 l |

Suspend 190 g β-Cyclodextrin in 2.0 l Ethanol 95%, mix intensively in a blender with 5 kg of wet MCC, pass through a sieve (0.5 mm) and dry the material at 80°C. Pass again through a sieve of 0.250 mm.
III

| | |
|---|---|
| MCC | 5.0 kg |
| β-Cyclodextrin | 1.25 kg |
| Water | 4.5 kg |

The stirred suspension of β-Cyclodextrin in water is sprayed continuously in fluidized bed granulator on MCC. The inlet air temperature was about 60°C.
The drying process is finished, when the water content is below 6% in the final product. The material is then sieved through 250 micron screen.

The mixtures of the following compositions were prepared by the same methods.
1.

| | |
|---|---|
| MCC | 90 parts |
| β-Cyclodextrin | 10 parts |

2.

| | |
|---|---|
| MCC | 85 parts |
| β-Cyclodextrin | 15 parts |

3.

| | |
|---|---|
| MCC | 70 parts |
| β-Cyclodextrin | 30 parts |

4.

| | |
|---|---|
| MCC | 60 parts |
| β-Cyclodextrin | 40 parts |

5.

| | |
|---|---|
| MCC | 75 parts |
| β-Cyclodextrin | 25 parts |

6.

| | |
|---|---|
| MCC | 80 parts |
| α-Cyclodextrin | 20 parts |

7.

| | |
|---|---|
| MCC | 80 |
| γ-Cyclodextrin | 20 parts |

The resulting products have the following properties:

| | |
|---|---|
| Angle of repose: | 38 - 55° |
| Bulk density: | 278 - 470 g/l |
| Particle size: | > 75 µm max. 75% |
| distribution: | > 250 µm max. 1%. |

### Tabletting examples:

A)

| | |
|---|---|
| Mixture of Ex. I | 99.5 parts |
| Lubricant magnesium stearate | 0.5 part |

The components are mixed for 5 minutes and then converted in a rotary tabletting machine into biplanar tablets of diameter 12 mm, weighing 500 mg, with a moderate pressure (5-10 kN). The resulting tablets have a hardness of 180-290 N and a disintegration time of 5 min.
Compared to that a physical mixture of the said powdered components proves to be distinctly less satisfactory than the novel tabletting auxiliary.
The loading capacity is important for direct tabletting auxiliaries. The substances preferably used for testing the loading are those which are known to be difficult to tablet such as paracetamol or acetylsalicyclic acid. Besides high loading, also important are good disintegration properties, hardness and friability.
B) The tabletting is carried out as described under A) using

| | |
|---|---|
| Paracetamol | 350 parts |
| Mixture of Ex. I | 148 parts |
| Stearic acid powder | 2 parts |

The loading is thus 70%, but loading up to 80% is possible.

| | |
|---|---|
| Weight: | 500 mg |
| Hardness: | 115 N |
| Disintegration: | 1 - 2 min |
| Friability: | 0.5% |

Comparison with direct tabletting using Avicel® pH 101 (MCC from FMC) yields the following results:

| | |
|---|---|
| Weigth: | 500 mg |
| Hardness: | 95 N |
| Disintegration: | 5 min |
| Friability: | 0.3% |

Thus a lower pressure is required on use of the novel direct tabletting auxiliaries to obtain the same tablet hardness.
C) The procedure is as in Example A), using

| | |
|---|---|
| Acetylsalicylic acid | 400 parts |
| Mixture of Ex. I | 60 parts |
| Potato starch | 35 parts |
| Stearic acid powder | 5 parts |

Corresponding to a loading of 80%

| | |
|---|---|
| Weight: | 500 mg |
| Hardness | 97 N |
| Disintegration: | 5 min |
| Friability: | 0.2% |
| Diameter: | 12 mm |

Comparison of the direct tabletting with MCC in place of mixture 5 yields the following results:

| | |
|---|---|
| Weight: | 500 mg |
| Hardness: | 72 N |
| Disintegration | 5-10 min |
| Friability: | 0.3% |
| Diameter: | 12 mm |

## Claims

1. A direct tabletting auxiliary comprising on a weight basis
(a) 60 to 98% of a filler comprising microcrystalline cellulose or cellulose powder, and
(b) 2 to 40% of a binder comprising α-, β-, or γ-cyclodextrin, characterised in that the auxiliary is formed by mixing (a) and (b) with water or water/alcohol and then drying the mixture.

2. A direct tabletting auxiliary as claimed in claim 1, wherein
(a) the filler is present in an amount of 80 to 98%, and
(b) the binder is present in an amount of 2 to 20%.

3. A direct tabletting auxiliary as claimed in claim 1, wherein the binder is β-cyclodextrin.

## Patentansprüche

1. Direkter Tablettierhilfsstoff, umfassend auf einer Gewichtsbasis
(a) 60 bis 98% eines Füllstoffs, umfassend mikrokristalline Cellulose oder Cellulosepulver, und
(b) 2 bis 40% eines Bindemittels, umfassend α-, β- oder γ-Cyclodextrin, dadurch gekennzeichnet, daß der Hilfsstoff durch Mischen von (a) und (b) mit Wasser oder Wasser/Alkohol und anschließendem Trocknen des Gemischs hergestellt wird.

2. Direkter Tablettierhilfsstoff nach Anspruch 1, worin
(a) der Füllstoff in einer Menge von 80 bis 98% vorhanden ist, und
(b) das Bindemittel in einer Menge von 2 bis 20% vorhanden ist.

3. Direkter Tablettierhilfsstoff nach Anspruch 1, worin das Bindemittel β-Cyclodextrin ist.

## Revendications

1. Auxiliaire de compression directe comprenant, en pourcentage pondéral,
(a) 60 à 98 % d'une charge comprenant de la cellulose microcristalline ou de la cellulose en poudre, et
(b) 2 à 40 % d'un liant comprenant de la α-, β- ou γ-cyclodextrine, caractérisé en ce que l'auxiliaire est formé en mélangeant (a) et (b) avec de l'eau ou avec un mélange eau/alcool puis en séchant le mélange obtenu.

2. Auxiliaire de compression directe selon la revendication 1, caractérisé en ce que
(a) le pourcentage de charge est compris entre 80 et 98 %, et en ce que
(b) le pourcentage de liant est compris entre 2 et 20 %.

3. Auxiliaire de compression directe selon la revendication 1, caractérisé en ce que l'agent liant est la β-cyclodextrine.
